# EUROPEAN PATENT APPLICATION

(11) **EP 1 321 529 A1**
(43) Date of publication of application: **25.06.2003**
(21) Application number: 01130640.4
(22) Date of filing: 21.12.2001
(51) Int. Cl.: C12Q 1/48, G01N 33/60, G01N 33/50, G01N 33/573

(54) **Methods for identifying compounds that modulate sister chromatid cohesion**

(71) Applicant: BOEHRINGER INGELHEIM INTERNATIONAL GmbH, 55218 Ingelheim am Rhein (DE)
(72) Inventor: Eisenhaber, Frank, A-1210 Wien (AT); Schleiffer, Alexander, A-1200 Wien (AT); Ivanov, Dimitri, A-1100 Wien (AT); Nasmyth, Kim, A-1010 Wien (AT)
(74) Representative: Laudien, Dieter, Dr.

(57) **Abstract**

Method for identifying compounds which modulates cohesion of sister chromatids by modulating the acetyltransferase activity of Eco1

## Description

The invention relates to compounds influencing mitosis and meiosis in eukaryotic cells and methods for identifying such compounds. In particular, the invention largely relates to the treatment and prevention of human conditions by modulating sister chromatid cohesion.

Cohesion between sister chromatids is established during S-phase and maintained through G2 phase until it is resolved in anaphase [for review see [1-3]]. In *Saccharomyces cerevisiae*, a complex consisting of Scc1, Smc1, Smc3 and Scc3 proteins, called cohesin, mediates the connection between sister chromatids [4-6]. The evolutionary conserved yeast protein Eco1 is required for establishment of sister chromatid cohesion during S-phase but not for its further maintenance during G2 or M phases or for loading the cohesin complex onto DNA [6, 7]. However, no similarity of Eco1 to the proteins of known function has been reported and the mechanism of its action remained completely unknown.

It was an object of the invention to analyze the molecular functions of Eco 1 in order to utilize Eco 1 as a target for therapy, in particular for cancer therapy, and as a target in all other situations where modulation of sister chromatid cohesion is therapeutically or otherwise beneficial.

To solve the problem underlying the invention, sensitive sequence analytic techniques, including hidden Markov model domain fragment searches, were used to define the functions of Eco1. In the present invention, it was found that Eco1 posesses a novel acetyltransferase activity that was first predicted *in silico* and then confirmed biochemically. Of note, Eco1 has only a marginal degree of similarity to the known GNAT superfamily acetyltransferases that had escaped previous database searches. The invention provides the first successful case of non-trivial function assignment for an uncharacterised protein with a hidden Markov model domain fragment search. Eco 1 was found to be a two-domain architecture with an N-terminal C₂H₂ Zn finger-like domain and a ∼150 residue C-terminal domain with an apparent acetyl coenzyme A binding motif. Eco 1 acetyltransferase is active in an *in vitro* assay towards a variety of cohesion-related substrates. However, it has no detectable activity towards histones. While this raises the question of specificity of the modification observed *in vitro,* it suggests that an *in vivo* target of the new enzyme will most likely be a non-histone protein. The fact that the G211D mutation that causes temperature-sensitivity *in vivo* (i) is located in the most conserved region that is characteristic for known acetyltransferases and responsible for coenzyme A binding and (ii) triggers a dramatic reduction of enzymatic activity *in vitro* strongly suggests that Eco 1 functions as an acetyltransferase *in vivo* as well and the disruption of this activity causes the mutant phenotype. The data of the present invention suggest that establishment of sister chromatid cohesion is regulated, directly or indirectly, by an acetyltransferase.

These findings provide the prerequisite for interfering with the function of Eco 1 in the mechanism of establishing and maintaining sister chromatid cohesion and thus a novel approach for inhibiting the proliferation of rapidly dividing animal cells, in particular tumor cells.

Thus, the present invention relates to screening methods and assays for agents which modulate, i.e. disrupt, inhibit or enhance the acetyltransferase activity of Eco1 and/or modulate interaction with and/or acetylation of the Eco1 substrates by Eco1, thereby affecting cellular processes in which Eco 1 is involved.

In a general aspect, the present invention relates to a method for identifying a compound which modulates cohesion of sister chromatids by modulating the acetyltransferase activity of Eco1 including:
(a) incubating an Eco1 polypeptide that has an acetyltransferase activity in the presence of an Eco 1 substrate with a test compound; and
(b) determining acetyltransferase activity of said Eco 1 polypeptide.

A principle of an acetylation assay, according to which the screening assay of the invention may be conducted, is described in Example 2, where recombinant Eco1 was incubated in the presence of a labeled acetyl coenzyme A (¹⁴C- acetyl coenzyme A) and the transfer to the label to the substrate is detected.

Preferably, the Eco1 polypeptide employed in the method of the invention is the full-length polypeptide; however, a fragment thereof that retains the acetyltransferase activity of the wildtype Eco 1 protein may also be used.

Eco1 substrates useful in the assay may be those equivalent to or mimicking the naturally occuring substrates, e.g. crude chromatin preparations or Scc1, Scc3, and Pds5, which were shown to be substrates for Eco1 in vitro (see Example 2).

For use in the method of the present invention, the Eco1 substrate may be the full-length protein, a truncated portion or fragment, or a truncated portion fused to another protein (eg. GST), or a suitable variant, allele, mutant analogue or derivative of any of these.
Preferably, the human substrate homologs are employed.

Alternatively, substrate peptides may be used that contain the acetylation site of the substrate. To obtain suitable peptide substrates, peptides (with random sequence) are spotted onto a solid support surface and incubated with the Eco1 enzyme in the presence of radioactive AcCoA. Peptides suitable as Eco1 substrates will light up by the radioactive acmark. These peptides represent artificial substrates; however statistical analysis of the peptide sequence combined with data base mining may identify physiological substrates. Suitable technology for this approach is available from Fa. Jerini, BRD).

In a preferred embodiment, the substrate employed in the screening method of the invention is an *in vivo* substrate of Eco1.

To obtain the in vivo substrate(s) of Eco1, preferably an *in vivo* approach is applied that uses radioactive labeling of cells with tritiated acetate followed by 2D gel-electrophoresis. One can compare wild type and Eco1 mutant cell preparations and identify the spots that are dependent on Eco 1 function.

Alternatively to the above-described in vivo approach, cellular extracts, fractionated cellular extracts, subfractions, etc. can be separated on (native) gels and incubated with Eco1 and radioactive AcCoA (in-gel assays). Radioactive bands are excised from the gel and analysed to identify the radio-acetylated proteins (i.e. Eco1 target proteins). Suitable methods employ 2D gels, MALDI-TOF, microsequencing etc. Example of such in-gel activity assays were described by Brownell and Allis (1995), *Proc. Natl. Acad.* Sci., **92:** 6364-6368 or Mizzen, et al (1996), Cell, **87:** 12611270.

These in vitro in gel assays or assays with selected recombinant substrates or screenings of the protein libraries (available for yeast as an array of gst-fusions) will generate a relatively great number substrates that will be retested in vivo by either radioactive labelling and immunoprecipitation or by immunoprecipitation followed by Western blotting with anti-acetyl-lysine antibodies.
The protein components used in the assay method of the invention, i.e. Eco1 and the substrate proteins, or fragments thereof, respectively, can be produced recombinantly according to standard methods, e.g. in bacteria or in yeast or insect cells or in other suitable host cells, based on the sequence information in the literature or in databases.

The sequences for Eco1 and the substrates, respectively, are available from databases under the following accession numbers:
Eco1:
   Eco1_Sc [Saccharomyces cerevisiae]:NCBI protein Acc Nr.: NP_011218
   Eco1a_Hs [Homo sapiens]: Genebank Acc Nr.: BG288675
   Eco1b_Hs [Homo sapiens]: Genebank Acc Nr.: XM_056302
Substrate proteins:
   Scc1:
      Scc1_Sc [Saccharomyces cerevisiae]: NCBI protein Acc. Nr.: NP_010281
      Scc1_Hs [Homo sapiens]: Genebank Acc. Nr. NM_006265
   Scc3:
      Scc3_Sc [Saccharomyces cerevisiae]. NCBI protein Acc. Nr.: NP_012238
      SA-1_Hs [Homo sapiens]: Genebank Acc. Nr.: NM_005862
      SA-2_Hs [Homo sapiens]:Genebank Acc. Nr.: NM_006603
      SA-3_Hs [Homo sapiens]: Genebank Acc. Nr.: NM_012447
   PDS5:
      Pds5_Sc [Saccharomyces cerevisiae]: NCBI protein Acc. Nr.: NP_013793
      Pds5_Hs [Homo sapiens]: Genebank Acc. Nr.: NM_015032

In the case of EST clones being available, full length cDNAs can be isolated according to the methods described below from human libraries, and analysed to determine whether they correspond to the respective human homologues.

Alternatively to the identification of human homologues by means of information obtained from public sequence databases, human homologues can be directly isolated by a low stringency PCR amplification from human RNA extracted from certain tissues, using standard PCR protocols. Degenerate oligonucleotides can be designed according to standard protocols to match regions of high sequence in the proteins. These oligonucleotides can then be used to amplify candidate human homologues from RNA prepared from selected tissues. Amplified products can then be cloned into suitable vectors for propagation in bacteria, and the DNA sequence of individual amplification products determined and used to identify human homologues according to the criteria defined above.

Alternatively, human homologues may be obtained from a cDNA library prepared directly from such RNA, using routine molecular biology methods (*see*, *e.g.*, *Molecular Cloning*, *A Laboratory Manual*, 2nd Ed., Sambrook, J., *et al.*, eds., Cold Spring Harbor, New York: Cold Spring Harbor Laboratory Press (1989). Since determined nucleotide sequences are provided in databases, generating polynucleotides which hybridize to a portion of the cDNA molecule would be routine to the skilled artisan.

The obtained protein can be purified by conventional biochemical fractionation from yeast cells over-producing Eco 1 or by tagging the over-produced protein with polypeptide sequences which have a special affinity for a defined ligand (affinity purification). For example, Eco 1 can be purified on nickel-agarose columns if it has been tagged with multiple histidine residues, whereas it can be purified on glutathione-agarose columns if it has been tagged with GST. Such affinity purification involves the cleavage of Eco1 from its tag using site specific proteases or self cleaving inteins.

Methods for successful production of acetyltransferases in bacteria have been described in the literature, e.g. by Franklin and Clarke, 2001; they can be easily adapted to Eco1.

The acetyltransferase activity may be assayed by any of a variety of procedures as discussed below and may be readily adapted to high throughput scale.

For example, the substrate may be immobilized, e.g. on a bead or plate, and acetylation detected using an antibody or another binding molecule which binds to the relevant acetylation site with an affinity that depends on the acetylation. Such antibodies are commercially available (e.g. the anti-acetyl antibody purchased from NEB that was used in Example 2) or may be obtained by any standard technique, e.g. using as an antigen an acetylated peptide (such as a fragment of Scc1). Binding of the antibody, which discriminates between the acetylated and non-acetylated form of the substrate, may be assessed using any technique available to those skilled in the art, which may involve determination of the presence of a suitable label.

Acetylation may also be assayed in solution, e.g. as described in Bannister and Kouzarides (1996), *Nature*, **384:** 641-643. Briefly, the substrate and acetyltransferase are mixed, and the acetyleaction reaction is initiated by the addition of labeled acetyl CoA (e.g.¹⁴-C - acetyl CoA) and incubated at 30°C for 10-45 min. The reaction products are then resolved by SDS-PAGE and viewed following fluorography of the gel. Alternatively, following SDS-PAGE, the resolved proteins can be Western blotted to a nitrocellulose membrane, which is then dried and exposed to film.

A further example for a suitable assay to monitor the effect of a test compound on Eco1 actetyltransferase activity is a fluorescent method that is based on the phenomenon of fluorescence resonance energy transfer (FRET), as described by Gershkovich et al., 1996 or by Matayoshi et al., 1990. This assay employs a substrate peptide containing a single lysine as an acetylation site and carrying two terminal fluorophores that constitute a FRET pair. This substrate peptide can be cleaved by an endopeptidase, e.g. Lys-C from Lysobacter enzymogenes (which is commercially available e.g. from Roche Molecular Biochemicals), that cleaves the substrate specifically after lysine, but only in its native, non-acetylated form. Cleavage of the non-acetylated peptide by the protease releases two peptides, one of them showing fluorescence properties since its fluorophore is not quenched, thereby a fluorescence signal is generated, which can be monitored by fluorometric spectrometry. The presence of Eco1, which acetylates the peptide, reduces or abolishes the signal. A test compound that inhibits the acetyltransferase activity of Eco1 can be identified by an increased signal value.

Suitable control experiments employ assay conditions under which full acetylation is obtained (negative control) or conditions in which a non-acetylated peptide is incubated with the protease to obtain a maximal signal.

The test compounds may be natural or synthetic chemical compounds that are available in compound libraries. Also, extracts of plants, microbes or other organisms may be used. An alternative source of test compounds are combinatorial chemical libraries.

In a further aspect, the invention relates to a pharmaceutical composition which contains, as the active ingredient, one or more compounds which interfere with chromatid cohesion by inhibiting the acetyltransferase activity of Eco1.
In a preferred embodiment, the invention comprises pharmaceutically active compounds and their use in therapy, which are small chemical molecules that have been identified as Eco1 inhibitors in the screening method of the invention.

Alternatively, the Eco1 inhibitors may be biological molecules, e.g. peptides or peptide-derived molecules like peptidomimetics.

The efficacy of compounds identified as Eco1 inhibitors can be tested for *in vivo* efficacy either on yeast cells or in mammalian cells. Effective compounds should block (or at least in some way interfere with) sister chromatid cohesion, which can be measured, e.g. by using CenV-GFP in yeast, as described by Ciosk et al., 1998, or standard cytological techniques in mammalian cells.

Effective compounds should be either cytostatic or cytotoxic. Substances whose potential for therapeutic use has been confirmed in such secondary screen can be further tested for their effect on tumor cells. To test the inhibition of tumor cell proliferation, primary human tumor cells are incubated with the compound identified in the screen and the inhibition of tumor cell proliferation is tested by conventional methods, e.g. bromo-desoxy-uridine or ³H incorporation. Compounds that exhibit an anti-proliferative effect in these assays may be further tested in tumor animal models and used for the therapy of tumors.

Further suitable secondary assays for validating the identified compounds are the Alamar Blue Assay, a spectrophotometric microassay using Alamar Blue dye, assays measuring apoptosis, e.g. the TUNEL assay, assays identifying effects on the cell cycle, in particular arrest in G₂M phase, using FACS analysis, or biochemical assays determining the acetylation of the in vivo key substrates (cohesin) of Eco1.

Toxicity and therapeutic efficacy of the compounds identified as drug candidates by the method of the invention can be determined by standard pharmaceutical procedures, which include conducting cell culture and animal experiments to determine the IC₅₀, LD₅0, the ED₅₀. The data obtained are used for determining the human dose range, which will also depend on the dosage form (tablets, capsules, aerosol sprays, ampules, etc.) and the administration route (oral, buccal, nasal, paterental or rectal). A pharmaceutical composition containing the compound as the active ingredient can be formulated in conventional manner using one or more physologically active carriers and excipients. Methods for making such formulations can be found in manuals, e.g. "Remington Pharmaceutical Sciences".

Influencing sister chromatid cohesion may be also beneficial in preventing birth defects caused by missegration of chromosomes in human meioses. For example, since cases of human aneuploidy such as Down's syndrome may be caused by premature separation of sister chromatids (Griffin, 1996), the use of a drug that enhances Eco1 activity might be able to reduce precocious sister separation and thereby the incidence of aneuploidy in human fetuses.

Thus, in a further aspect, the invention relates to compounds upregulating Eco1 activity for the prevention of birth defects caused by missegration of chromosomes in human meioses.

### Brief description of the drawings:

### Figure 1.

### A. Domain architecture of Eco1 from Saccharomyces cerevisiae.

### B. Three-dimensional structure of GCN5 histone N-acetyltransferase from Tetrahymena thermophilum (1QSN) [20].

The labeling of the structural elements is according to the sequence alignment in A.

### Figure 2.

### A. Eco1 autoacetylates in vitro.

Wild type and mutant Eco1 proteins were expressed as GST-fusions in bacteria. Increasing amounts of Eco1 were incubated in the presence of ¹⁴C-acetyl coenzyme A and separated on SDS-PAGE. Coomassie stain (upper panel) and phosphorimager-scan (lower panel) of the same gel are shown. Eco1 band on Coomassie-stained gel is indicated with an arrow and a non-specific band with an asterisk. Although not clearly visible on the gel, the same non-specific band is present in the wild type preparation but is less abundant compared to the Eco1 protein.

### B. Autoacetylation of Eco1 is detected by an acetyl-lysine antibody.

Wild type Eco1 protein was incubated either alone (lane 1) or in the presence of the increasing concentrations of cold acetyl coenzyme A (lanes 2-5). Samples were separated on SDS-PAGE and Western blot analysis with an anti-acetyl-lysine antibody (Ac-K-103, NEB) was performed. The background acetylation from the bacterial expression system (see text) is probably responsible for a weak immunoreactivity with anti-acetyl-lysine antibody in the absence of acetyl conenzyme A.

### C. Eco1 does not acetylate histones.

Eco1 or PCAF were incubated with ¹⁴C- acetyl coenzyme A either alone or in the presence of all bovine core histones (lanes 3-5) or histone H3 alone (lanes 6-8). Phosphorimager scan of an SDS-PAGE gel is presented.

### Figure 3.

### A. Scc1 is acetylated by Eco1 in vitro. (Left and middle panels).

*In vitro* acetylation assay was performed with cold acetyl coenzyme A and Eco1, full length Scc1 expressed in baculovirus or both proteins together. Same Western blot was analysed with anti-acetyl-lysine (left panel) or anti-His₅ antibody (middle panel). *(Right panel).* Same assay was performed with radioactive acetyl coenzyme A. Phosphorimager scan of SDS-PAGE gel is shown.

### B. Lysine 210 of Scc1 is modified by Eco1.

The middle fragment of Scc1 was expressed as Gst-fusion in bacteria. Either wild type or proteins with mutated lysines were used as substrates in an *in vitro* acetylation assay with Gst-Eco 1. Upper and middle panels show Coomassie-stain and phosphorimager scan of the same gel. The lower panel is the Western blot probed with an anti-acetyl-lysine antibody.

### C. Scc3 and Pds5 are also acetylated by Eco1 in vitro.

*In vitro* acetylation assays were performed with Gst-Eco1 and Scc3 and Pds5 proteins expressed in baculovirus. Western blot with anti-acetyl-lysine antibody and a phosphorimager scan of a radioactive assay are shown.

### Example 1

### Sequence analysis of Eco1

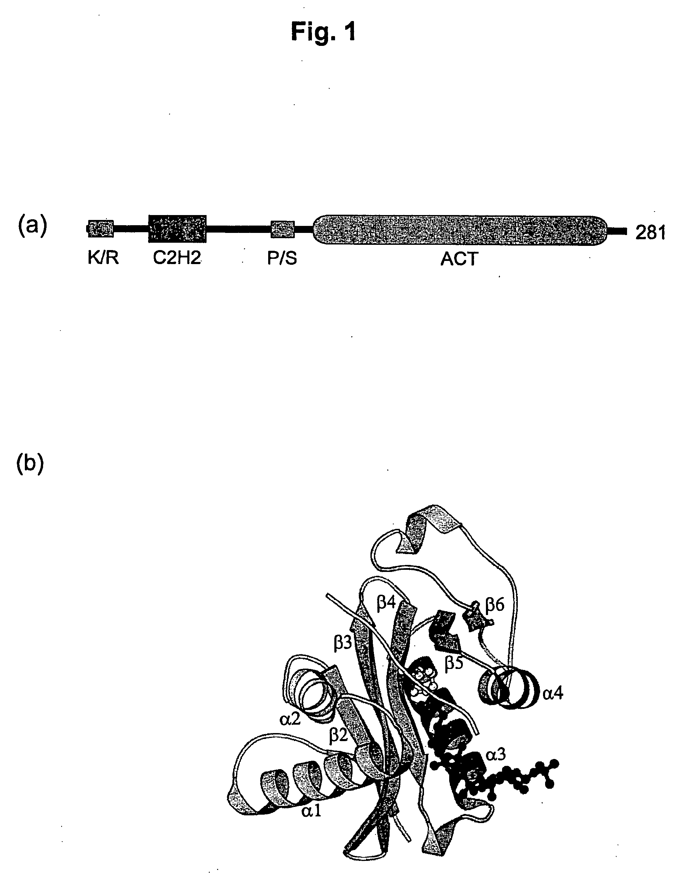

The Eco1 sequence is evolutionary conserved throughout eukaryotes and it has significant similarity to human and mouse ESTs [7]. However, no convincing suggestion of molecular function has yet been identified [7, 8]. In an attempt to extend previous results [6], the Eco1 protein sequence of *Saccharomyces cerevisiae* was subjected to a three-step sequence analysis procedure with bioinformatic methods. First, the invenors studied the lexical structure of its sequence and looked for compositionally biased regions and other typically non-globular protein segments. The inventors identified two polar regions of low complexity: one at the N-terminus (residues 1-13) rich in positive charges (totally 6) and another between sequence positions 95-107 with many prolines and serines, a probable linker region between two putative globular domains (Fig. 1A).

The Eco1 sequence was next compared with libraries of known sequence domains and motifs. The sequence segment 33-57 has a positive score (=0.9; but statistically not significant E-value >1) with the classical C₂H₂ Zn finger domain model of PFAM in the global domain search mode [6, 9]. The Eco1 sequence differs from the well-known PROSITE motif PS00028 "C-x(2,4)-C-x(3)-[LIVMFYWC]-x(8)-H-x(3,5)-H" by a two residue insertion in the loop spanning the cysteine and the histidine residues in the motif center (14 residues in loop 39-42 from Eco1 instead of 12 in the classical motif).

The inventors also found that the PFAM model PF00583 "Acetyltransferase (GNAT) family" matches region 212-236 of Eco1 in the local (domain fragment) search mode (score 12.1, E=0.009). Generally, such short fragment hits do not implicate any relationship with the protein sequence family represented by the HMM model if they are not substantiated by additional, non-statistical arguments (for example, structural considerations or biological context) [10]. In this case, the sequence piece is essentially identical to the part of the acetyltransferase model describing the motif A, the acetyl-coenzyme A binding region of the GNAT superfamily, and critical residues for ligand binding such as R222, G225, and D232 are well conserved [11, 12]. In addition to this functional argument, the inventors found highly similar secondary structure predictions among Eco1 proteins (see below, Fig.1).

Third, the inventors collected the family of sequences homologous to Eco1 with Blast/Psi-Blast [13, 14] and HMM [15] searches and found representatives for a number of eukaryotes including all heavily sequenced species among fungi, animals and plants. Both in the human and the mouse EST databases, 2 distinct Eco1 variants with different tissue-specific expression (as deduced from EST annotations) were detected.

All homologues of Eco1 in other species have also been subjected to the same three-step analysis procedure. The C₂H₂ Zn finger-like motif of yeast Eco1 was found in all apparently complete sequences except for the protozoan proteins from *Trypanosoma brucei* (emb1 CAB95441) and *Leishmania major* (emb1 AL499619, conceptual translation from genomic clone). Remarkably, a *Drosophila* acetyltransferase MOF that belongs to a MYST family of histone acetyltransferases characterized by the presence of C₂HC Zn finger close to their catalytic domain, is demonstrated to use the Zn finger to contact the nucleosome and the histone H4 N-terminal tail. Mutations in the Zn finger greatly diminish MOF activity *in vitro [16].*

Indications for an acetyltransferase domain, previously referred to as Eco1 domain [6], were also detected in all homologues. The amino acid sequence region 945-1008 of Eco1 from *Drosophila melanogaster* was the best hit in a domain fragment search with the PFAM model PF00583 (score 17.5, E-value 6.4e-05). In the case of the latter, even the complete PFAM domain model produces a hit with a positive score (but a non-significant E-value) for a segment comprising 98 residues. The inventors found that the N-terminus of the *S. pombe* Eco1 homologue is extended by more than 600 amino acids and the *D. melanogaster* homologue by 800 amino acids. In the first case, the N-terminal extension is the known fusion with polymerase η [6, 17] and contains a UMUC-domain known to be involved in DNA replication and UV protection [18]; in the fly protein case, no function could be guessed for this extension, which could also represent a genome assembly artefact.

Starting from the PF00583 model hits in Eco1 homologues and relying on the highly similar secondary structure predictions for the Eco1 proteins, a multiple alignment including the Eco1 sequences and acetyltransferases with a known 3D structure [12, 19, 20] was constructed and plausibly extended both in N- and C-terminal directions. The core alignment region comprises the sequence motifs D, A, and B, the β-strands 2-5 and the α-helix 3 and 4 of the GNAT fold (Fig. 1B). Apparently, Eco1 homologues lack the α-helix 2 of the histone acetyltransferase structure. Considering the low level of sequence conservation with GNAT superfamily, the Eco1 C-terminal domain represents a new sequence family.

To conclude, Eco1 protein sequence analysis results indicate a two-domain structure: 1) an N-terminal region with many positive charges (K/R) and a C₂H₂ Zn finger-like domain (residues 33-57, C2H2), possibly with a nucleic acid or protein binding function, 2) a proline- and serine-rich low complexity region (residues 95-107, P/S), the probable inter-domain linker, and 3) a C-terminal domain with suggested acetyltransferase activity (residues 111-266, ACT) (Fig. 1A).

### Example 2

### Biochemical tests

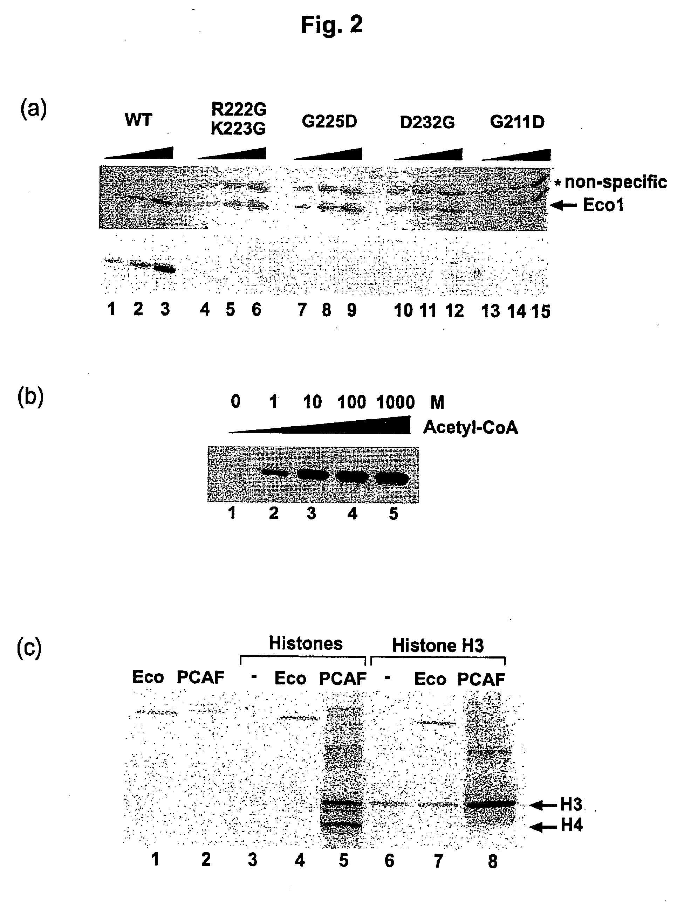

In order to test whether Eco 1 has an enzymatic activity *in vitro,* the inventors expressed the yeast protein as a GST-fusion in *E. coli.* When this recombinant protein was incubated in the presence of ¹⁴C- acetyl coenzyme A, transfer of label to a protein was readily detectable (Fig. 2A, lanes 1-3). To confirm that this was not due to a very tight binding of Eco1 to acetyl coenzyme A, Eco 1 was incubated with a cold substrate and then analyzed by Western blot with a commercial anti-acetyl-lysine antibody (NEB) (Fig. 2B). This antibody recognizes an epitope comprised of an acetyl group covalently coupled to the amino group of lysine side chain. Eco1 incubated in the absence of acetyl coenzyme A in the control reaction was detected by this antibody only weakly (lane1). However, upon addition of increased amounts of the substrate the intensity of the band detected by the Western blot greatly increased (lanes 2-5). This indicated that the reaction is accompanied by a covalent transfer of the acetyl group to a lysine residue of the protein. Eco 1 was further analyzed by mass-spectrometry to identify the acetylated amino acid residues. Surprisingly, Eco1 purified from bacteria was found to be already acetylated on at least 7 lysines. This is most likely due to autoacetylation occurring inside the cell in the process of expression.

Transfer of an acetyl group from acetyl coenzyme A to a primary amine in aqueous solution is an energetically favorable reaction resulting in detectable rates of nonenzymatic background acetylation [21]. To rule out this possibility, the inventors decided to confirm that the observed *in vitro* modification of Eco1 is dependent on its intact structure. The inventors mutated a number of amino acids conserved among Eco1 homologues from different organisms and tested the mutant forms of enzyme for activity *in vitro.* The mutation leading to a temperature-sensitive allele *of eco1* was identified as glycine 211 to aspartic acid substitution [6]. In our model of Eco1 fold, this position corresponds to the fourth residue in the α4 helix in the motif A, the longest and most highly conserved motif among the members of GNAT superfamily of acetyltransferases. Strand β4 and the following helix α3 form the core of the acetyl coenzyme A binding site [12]. Remarkably, a mutation in Eco1 homologue of *S. pombe*, Esol, that was responsible for temperature-sensitivity, was an identical glycine to aspartic acid substitution at position 799 corresponding in the alignment to position 211 of Eco1 [17]. Another mutation that the inventors used in Eco1 was a glycine to aspartic acid substitution at amino acid 225. This position corresponds to glycine 691 in a *Drosophila* histone acetyltransferase MOF. Substitution of this glycine by a glutamic acid resulted in the absence of MOF activity *in vivo* [22]. Similarly, mutation of corresponding glycine 104 in the human spermidine/spermine acetyltransferase to aspartic acid resulted in loss of activity [23]. Two more mutations that were generated were arginine 222/lysine 223 to glycine/glycine and aspartic acid 232 to glycine. In the predicted secondary structure, all mutated amino acids are located within motif A either in the helix α3 or immediately preceding it. When the wild type and mutant forms of Eco 1 were tested for self-acetylation in an *in vitro* radioactive assay (Fig. 2A), only a wild type enzyme was labeled efficiently (lanes 1-3). Mutant forms of Eco1 had a greatly reduced activity (lanes 4-15) that was weakly detected after the longer exposure times. This result demonstrates that an Eco 1 intact structure is required for the transfer of an acetyl group and that this is a *bona fide* enzymatic reaction.

A majority of confirmed protein acetyltransferases are capable of modifying histone tail domains. Therefore, the inventors tested whether Eco1 could use histones as a substrate. Bovine histone H3 alone (Fig. 2C, lanes 6-8) or a mixture of all 4 core histones (lanes 3-5) were incubated with ¹⁴C-acetyl coenzyme A either alone or in the presence of Eco1 or recombinant PCAF, a known histone acetyltransferase, expressed in bacteria that was used as a positive control. As expected, a measurable level of apparently nonenzymatic acetylation of histone H3 occurred in the absence of any added enzyme (lane 6). However, the addition of PCAF resulted in a greatly increased acetylation of its primary targets, histones H3 and H4 [24] (lanes 5 and 8), while the addition of Eco1 had no detectable effect (lanes 4 and7).

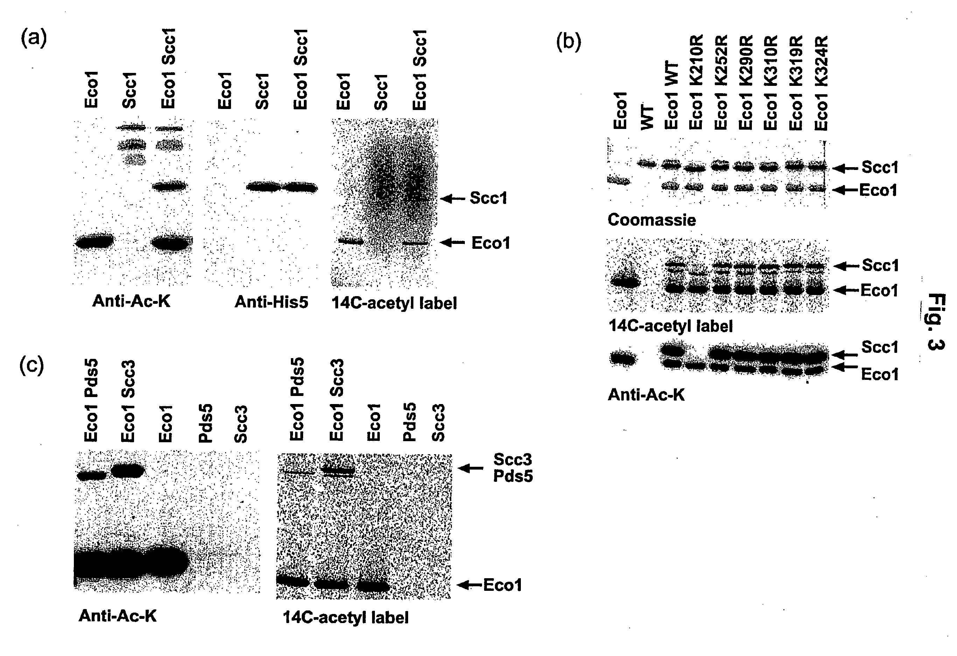

Next, the inventors assayed whether Eco1 can modify known components of the cohesin complex. First, yeast Scc1 protein was expressed in baculovirus system and incubated with cold acetyl coenzyme A and Eco 1 followed by a Western blot analysis with an acetyl-lysine antibody. Incubation of Scc1 in the presence but not in the absence of Eco1 resulted in a detectable degree of acetylation (Fig. 3A, left panel). The identity of the band was confirmed by probing the same Western Blot with an anti-His₅ antibody recognizing a poly-histidine tag on the recombinant Scc1 (Fig. 3A, middle panel). Scc1 was also labelled in a radioactive assay although weakly (Fig. 3A, right panel). In addition, the inventors observed Eco1-dependent acetylation of the *S. pombe* Scc1 homologue, Rad 21 (data not shown).

*S. cerevisiae* Scc1 contains 31 lysines. Based on the available prediction of its domain structure (unpublished results), Scc1 can be divided into 3 regions. The N-terminal and the C-terminal regions are likely to fold into defined domains with globular structure and the middle portion encompassing the separase cleavage sites presumably constitutes an unstructured linker region. To map the acetylation site, the inventors generated three fragments of Scc1 corresponding respectively to amino acids 1-168, 169-337 and 338-566, and expressed them as GST-fusions in *E.coli.* Only the middle portion of Scc1 was acetylated in the *in vitro* assay. This region contained only six lysines. They were subsequently mutated to arginines and the resulting mutants were assayed for acetylation by Eco1 (Fig. 3B). Mutating lysine 210 to arginine resulted in loss of acetylation. Mutating other lysines had no effect on the ability of the middle portion of Scc1 to be modified by Eco1 either in radioactive assay or by Western Blot analysis with anti-acetyl-lysine antibody. In addition, acetylation of lysine 210 was confirmed by mass-spectrometry analysis (data not shown).

The inventors tested whether other subunits of the cohesin complex can be acetylated by Eco1 *in vitro.* Scc3 and Pds5 proteins were expressed in baculovirus and tested as substrates in the *in vitro* assay. All of them were acetylated (Fig. 3C) with Scc3 demonstrating the highest degree of modification. The acetylation sites in Scc3 were further mapped by deletion analysis. Fragments containing amino acids 1-174, 175-350 and 961-1150 were acetylated while the middle portion of Scc3 was not. Mass-spectrometry analysis of the acetylated fragments identified the modified amino acids as lysines 13, 36, 78, 106, 224, 1071, and 1086.

The analysis of the *in vivo* role of a new enzymatic activity is clearly not a trivial task. For example, it took three years to demonstrate the galactosyltransferase activity of Fringe [25-27] after its prediction based on sequence considerations. To test whether any of the proteins involved in cohesion are acetylated *in vivo*, the inventors performed immunoprecipitation of the myc-tagged candidate proteins from yeast under denaturing conditions followed by Western blot analysis with commercially available anti-acetyl-lysine antibodies or a specific polyclonal antibody that was raised against a branched acetylated peptide corresponding to the region of Scc1 spanning lysine 210. In case of Scc1 and Scc3 the inventors matched the quantity of the immunoprecipitated protein to the recombinant protein used in the *in vitro* acetylation assay. Although acetylation of the recombinant protein was readily detected, there was no acetylation of the immunoprecipitated protein. A weak immunoreactivity that was observed with some of the proteins after long exposure times or using high antibody concentration was likely non-specific since there was no difference between protein precipitated from wild type strain and eco 1 mutant strain grown at restrictive temperature. In spite of our efforts, the inventors have been unable to detect acetylation of cohesin subunits, Pds5, and two other proteins implicated in cohesion, PCNA and Ctf18, *in vivo.* To further test whether acetylation of Scc1 has an *in vivo* function, the inventors constructed a yeast strain containing a wild type copy of Scc 1 driven by a galactose-inducible promoter and a mutant copy with lysine 210 substituted for arginine under a normal Scc1 promoter. This strain was viable when grown on medium with glucose as a single carbon source and showed no obvious growth or morphological defects. Considering that Eco1 function is essential in *S. cerevisiae*, this observation implies that if Scc1 acetylation is indeed occurring *in vivo* it is unlikely to be the only essential Eco1 substrate. This presently leaves the question of what is the crucial Eco1 substrate(s) *in vivo* unanswered. It still remains possible that one or more of cohesin subunits are acetylated in the process of establishment of cohesion but this modification is transient and the acetyl moiety is removed by deacetylases present on the chromatin. Alternatively the specificity of the available anti-acetyl-lysine antibodies may not allow detection of acetylation of these particular proteins. Radioactive *in vivo* labelling has been employed in the past for the detection of acetylation of more transient nature as is the case with p53 [28]. However, it also required overexpression of the protein in cells. Attractive candidates for acetylation by Eco1 are DNA polymerases themselves, such as Trf4 and DNA polymerase ε. The first was demonstrated to be specifically required for the establishment and perhaps also for maintenance of cohesion [29]. The second was shown to interact genetically with Eso1 in *S. pombe* [17]. Other possible targets might be components of DNA replication machinery or chromatin remodelling factors.

### References

1. Hirano T: Chromosome cohesion, condensation, and separation. *Annu Rev Biochem* 2000; 69:115-144.
2. Koshland DE, Guacci V: Sister chromatid cohesion: the beginning of a long and beautiful relationship. *Curr Opin Cell Biol* 2000; 12:297-301.
3. Nasmyth K, Peters JM, Uhlmann F: Splitting the chromosome: cutting the ties that bind sister chromatids. *Novartis Found Symp* 2001; 237:113-133.
4. Guacci V, Koshland D, Strunnikov A: A direct link between sister chromatid cohesion and chromosome condensation revealed through the analysis of MCD1 in S. cerevisiae. *Cell* 1997; 91:47-57.
5. Michaelis C, Ciosk R, Nasmyth K: Cohesins: chromosomal proteins that prevent premature separation of sister chromatids. *Cell* 1997; 91:35-45.
6. Toth A, Ciosk R, Uhlmann F, Galova M, Schleiffer A, Nasmyth K: Yeast cohesin complex requires a conserved protein, Eco1p(Ctf7), to establish cohesion between sister chromatids during DNA replication. *Genes Dev* 1999; 13:320-333.
7. Skibbens RV, Corson LB, Koshland D, Hieter P: Ctf7p is essential for sister chromatid cohesion and links mitotic chromosome structure to the DNA replication machinery. *Genes Dev* 1999; 13:307-319.
8. Jones S, Sgouros J: The cohesin complex: sequence homologies, interaction networks and shared motifs. *Genome Biol* 2001; 2.
9. Bateman A, Birney E, Durbin R, Eddy SR, Howe KL, Sonnhammer EL: The Pfam protein families database. *Nucleic Acids Res* 2000; 28:263-266.
10. Sander C, Schneider R: Database of homology-derived protein structures and the structural meaning of sequence alignment. *Proteins* 1991; 9:56-68.
11. Neuwald AF, Landsman D: GCN5-related histone N-acetyltransferases belong to a diverse superfamily that includes the yeast SPT10 protein. *Trends Biochem Sci* 1997; 22:154-155.
12. Dyda F, Klein DC, Hickman AB: GCN5-related N-acetyltransferases: a structural overview. *Annu Rev Biophys Biomol Struct* 2000; 29:81-103.
13. Altschul SF, Koonin EV: Iterated profile searches with PSI-BLAST--a tool for discovery in protein databases. *Trends Biochem Sci* 1998; 23:444-447.
14. Altschul SF, Madden TL, Schaffer AA, Zhang J, Zhang Z, Miller W, Lipman DJ: Gapped BLAST and PSI-BLAST: a new generation of protein database search programs. *Nucleic Acids Res* 1997; 25:3389-3402.
15. Eddy SR: Profile hidden Markov models. *Bioinformatics* 1998; 14:755-763.
16. Akhtar A, Becker PB: The histone H4 acetyltransferase MOF uses a C2HC zinc finger for substrate recognition. *EMBO Rep* 2001; 2:113-118.
17. Tanaka K, Yonekawa T, Kawasaki Y, Kai M, Furuya K, Iwasaki M, Murakami H, Yanagida M, Okayama H: Fission yeast Eso1p is required for establishing sister chromatid cohesion during S phase. *Mol Cell Biol* 2000; 20:3459-3469.
18. Sutton MD, Smith BT, Godoy VG, Walker GC: The SOS response: recent insights into umuDC-dependent mutagenesis and DNA damage tolerance. *Annu Rev Genet* 2000; 34:479-497.
19. Dutnall RN, Tafrov ST, Sternglanz R, Ramakrishnan V: Structure of the yeast histone acetyltransferase Hat1: insights into substrate specificity and implications for the Gcn5-related N- acetyltransferase superfamily. *Cold Spring Harb Symp Quant Biol* 1998; 63:501-507.
20. Rojas JR, Trievel RC, Zhou J, Mo Y, Li X, Berger SL, Allis CD, Marmorstein R: Structure of Tetrahymena GCN5 bound to coenzyme A and a histone H3 peptide. *Nature* 1999; 401:93-98.
21. Tanner KG, Trievel RC, Kuo MH, Howard RM, Berger SL, Allis CD, Marmorstein R, Denu JM: Catalytic mechanism and function of invariant glutamic acid 173 from the histone acetyltransferase GCN5 transcriptional coactivator. *J Biol Chem* 1999; 274:18157-18160.
22. Hilfiker A, Hilfiker-Kleiner D, Pannuti A, Lucchesi JC: Mof, a putative acetyl transferase gene related to the Tip60 and MOZ human genes and to the SAS genes of yeast, is required for dosage compensation in Drosophila. *Embo J* 1997; 16:2054-2060.
23. Lu L, Berkey KA, Casero RA, Jr.: RGFGIGS is an amino acid sequence required for acetyl coenzyme A binding and activity of human spermidine/spermine N1acetyltransferase. *J Biol Chem* 1996; 271:18920-18924.
24. Schiltz RL, Mizzen CA, Vassilev A, Cook RG, Allis CD, Nakatani Y: Overlapping but distinct patterns of histone acetylation by the human coactivators p300 and PCAF within nucleosomal substrates. *J Biol Chem* 1999; 274:1189-1192.
25. Yuan YP, Schultz J, Mlodzik M, Bork P: Secreted fringe-like signaling molecules may be glycosyltransferases. *Cell* 1997; 88:9-11.
26. Bruckner K, Perez L, Clausen H, Cohen S: Glycosyltransferase activity of Fringe modulates Notch-Delta interactions. *Nature* 2000; 406:411-415.
27. Moloney DJ, Panin VM, Johnston SH, Chen J, Shao L, Wilson R, Wang Y, Stanley P, Irvine KD, Haltiwanger RS, Vogt TF: Fringe is a glycosyltransferase that modifies Notch. *Nature* 2000; 406:369-375.
28. Gu W, Roeder RG: Activation of p53 sequence-specific DNA binding by acetylation of the p53 C-terminal domain. *Cell* 1997; 90:595-606.
29. Wang Z, Castano IB, De Las Penas A, Adams C, Christman MF: Pol kappa: A DNA polymerase required for sister chromatid cohesion. *Science* 2000; 289:774-779.
30. Gershkovich, A.A. and Kholodovych, V.V. (1996), J Biochem Biophys Meth 33, 135
31. Matayoshi, E.D. (1990), Science 247, 954
32. Franklin K, Clarke AJ. Antimicrob Agents Chemother 2001, Aug;45(8):2238-44

## Claims

1. A method for identifying a compound which modulates cohesion of sister chromatids by modulating the acetyltransferase activity of Eco1 including:
(a) incubating an Eco1 polypeptide that has an acetyltransferase activity in the presence of an Eco1 substrate with a test compound; and
(b) determining acetyltransferase activity of said Eco1 polypeptide.
